(19) Europäisches Patentamt
European Patent Office
Office européen des brevets

(11) Veröffentlichungsnummer: **0 196 039**
**A2**

(12) # EUROPÄISCHE PATENTANMELDUNG

(21) Anmeldenummer: **86103973.3**

(51) Int. Cl.⁴: **A61K 31/41**

(22) Anmeldetag: **22.03.86**

(30) Priorität: **29.03.85 DE 3511409**

(43) Veröffentlichungstag der Anmeldung:
**01.10.86 Patentblatt 86/40**

(84) Benannte Vertragsstaaten:
**AT BE CH DE FR GB IT LI NL SE**

(71) Anmelder: **BASF Aktiengesellschaft**
**Carl-Bosch-Strasse 38**
**D-6700 Ludwigshafen(DE)**

(72) Erfinder: **Janssen, Bernd, Dr.**
**Leuschnerstrasse 18a**
**D-6700 Ludwigshafen(DE)**
Erfinder: **Koenig, Horst, Prof. Dr.**
**Pariser Strasse 4**
**D-6700 Ludwigshafen(DE)**
Erfinder: **Scharwaechter, Peter, Dr.**
**Klinkerstrasse 26**
**D-2082 Moorrege(DE)**

(54) **Herstellung von Arzneimitteln gegen Viruserkrankungen.**

(57) Verwendung von 1-(4-Chlorphenyl)-1-(2,4-dichlorphenyl)-2-(1,2,4-triazol-1-yl)-ethan-1-ol (Formel I)

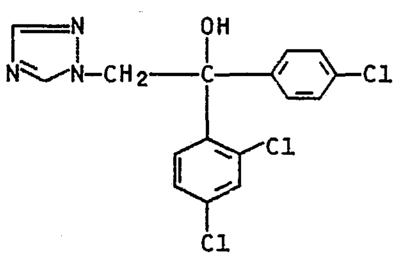

(I)

sowie seiner physiologisch verträglichen Salze bei der Herstellung von Arzneimitteln gegen Viruserkrankungen.

EP 0 196 039 A2

# Herstellung von Arzneimitteln gegen Viruserkrankungen

Die Erfindung betrifft die Verwendung von 1-(4-Chlorphenyl)-1-(2,4-dichlorphenyl)-2-(1,2,4-triazol-1-yl)-ethan-1-ol als Wirkstoff bei der Herstellung von Arzneimitteln zur Bekämpfung von Viruserkrankungen.

Es ist bekannt, daß Azolderivate wie 2-(α-Hydroxybenzyl)-benzimidazol (vgl. Burger's Medicinal Chemistry, IV. Ausg. 1979, Teil 2, S. 554 ff.: Hrsg. M. E. Wolff) und auch 2-Hydroxybutylazole wie Vibunazol (DE-OS 32 38 903), aber auch andere Vertreter dieser Stoffklasse (DE-OS 33 15 808) antivirale Wirksamkeit entfalten. Die Benzimidazole sind jedoch in vivo nicht wirksam, und auch die übrigen bekannten Verbindungen sind in ihrer Wirkung meist nicht befriedigend.

Von der Verbindung der Formel I ist bisher nur bekannt, daß sie fungizid (EP-PS 15 756) und antimykotisch - (EP-PS 36 153) wirkt.

Der Erfindung lag die Aufgabe zugrunde, einen neuen antiviralen Wirkstoff mit verbesserter Wirkung zu finden.

Es wurde nun gefunden, daß die Verbindung der Formel I sowie ihre physiologisch verträglichen Säureadditionssalze überraschenderweise sehr gute antivirale Eigenschaften aufweisen, und zwar bessere als die oben erwähnten, nach dem Stand der Technik bekannten Azolderivate. Die Erfindung besteht daher in der Verwendung von 1-(4-Chlorphenyl)-1-(2,4-dichlorphenyl)-2-(1,2,4-triazol-1-yl)-ethan-1-ol (Formel I)

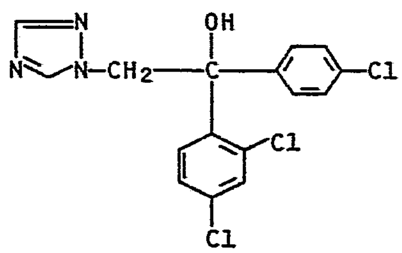

(I)

sowie seiner physiologisch verträglichen Salze bei der Herstellung von Arzneimitteln gegen Viruserkrankungen.

Die Verbindung der Formel I unterscheidet sich wesentlich u.a. durch die Art der Substituenten am Carbinolkohlenstoffatom von den oben genannten, bereits als antiviral wirksam beschriebenen Azolderivaten. Sie kann nach den in der EP-PS 36 153 angegebenen Verfahren hergestellt werden, indem man beispielsweise die Verbindung der Formel II,

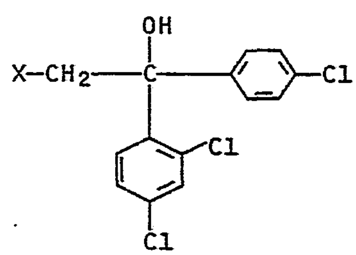

(II)

in der X eine nukleophil substituierbare Abgangsgruppe darstellt, mit 1,2,4-Triazol (III)

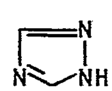

(III)

umsetzt und das so erhaltene Reaktionsprodukt gegebenenfalls in ein physiologisch verträgliches Säureadditionssalz überführt.

Als übliche Säuren zur Bildung physiologisch verträglicher Salze kommen vorzugsweise Halogenwasserstoffsäuren, wie Bromwasserstoffsäure, insbesondere Chlorwasserstoffsäure, mit der die erfindungsgemäße Verbindung ein besonders gut kristallisierendes Salz bildet, in Frage. Ferner sind zu nennen: Phosphorsäure, Salpetersäure, Schwefelsäure, mono-und bifunktionelle Carbonsäuren und Hydroxycarbonsäuren -wie Essigsäure, Maleinsäure, Bernsteinsäure, Fumarsäure, Weinsäure, Zitronensäure, Salicylsäure, Sorbinsäure, Milchsäure -, sowie Sulfonsäuren -wie p-Toluolsulfonsäure und 1,5-Naphthalindisulfonsäure -.

Die Verbindung der Formel I enthält ein chirales Zentrum und wird im allgemeinen als Racemat erhalten, aus welchem man mit den üblichen, bekannten Methoden -wie z.B. über die mit optisch aktiven Säuren gebildeten diaste-

reomeren Salze -einheitliche Enantiomere erhalten kann. Als antivirale Mittel kommen sowohl das Racemat als auch die einheitlichen Enantiomere sowie Gemische derselben in Betracht.

Wie bereits erwähnt, zeigt die Verbindung gemäß Formel I starke antivirale Aktivität, insbesondere gegen Herpes-Viren. Somit stellt die erfindungsgemäße Verwendung der Verbindung der Formel I eine wertvolle Bereicherung der Therapiemöglichkeiten bei Viruserkrankungen dar.

In den nachfolgenden Beispielen sind Testmethoden und antivirale Wirkungen von 1-(4-Chlorphenyl)-1-(2,4-dichlorphenyl)-2-(1,2,4-triazol-1-yl)-ethan-1-ol beschrieben. Als Vergleichswirkstoff diente das bekannte 2-(4-Chlorphenoxymethyl)-3,3-dimethyl-1-(1,2,4-triazol-1-yl)-2-butanol (Vibunazol).

Beispiel 1

Zellkulturversuche

Herpes Simplex Virus und menschliches Cytomegalovirus lassen sich in Kulturen von Affennierenzellen gut vermehren. Die Infektionen sind durch ca. 8 -12 h nach der Infektion auftretende charakteristische, mikroskopisch gut zu beobachtende und zu beurteilende cytopathische Effekte gekennzeichnet. Antivirale Eigenschaften von Testsubstanzen, die für die Wirtszellen nicht toxisch sind bzw. die ihre Wirkung gegen die infizierenden Viren in für die Zellen nicht toxischer Konzentration entfalten, können anhand der Reduktion des cytopathischen Effekts in diesem System gut charakterisiert werden.

Verwendet wurden Affennieren-Zellen des Stammes RC-37 Rita (Italodiagnostics, Rom), die in basalem Eagle's Medium (BME, Eagle, H., J. Exp. Med. 102, 595 -601, 1955) bis zur Infektion mit den HSV-1 Stämmen ANG - (Schröder et al., Intervirology 6, 270 -284, 1976) und WAL (Kirchner et al., J. Immunol. 117, 1753, 1978) gezüchtet wurden. Bei der Virusinfektion wurde das BME Medium gegen Medium 199 (Morgan et al., Proc. Soc. Exp. Biol. Med. 73, 1 -8, 1950) ausgetauscht. Infiziert wurden die Zellen nach Ausbildung eines fast-konfluenten Rasens mit einer Multiplizität von zwei infektiösen Viruspartikeln (PFU) pro Zelle. Die Virusstammsuspensionen hatten Titer zwischen 2 X $10^7$ und 1 X $10^8$ PFU/ml. Die Testsubstanzen wurden zu verschiedenen Zeiten zwischen 1 Stunde vor bis 2 Stunden nach der Infektion in nicht cytotoxischer Konzentration in das Medium der infizierten Zellen gegeben. Die Cytotoxizitätsgrenzen wurden anhand von Wachstumskurven und Koloniebildungsvermögen vereinzelter Zellen getestet. Die Wirkung der Substanzen wurde gegenüber Kontrollkulturen sowohl anhand mikroskopischer Beobachtung als auch durch Ermittlung der Zellnachkommenviren verfolgt. Die Virustiter wurden nach Russel (Nature, London, 195, 1028 -1029, 12962) bestimmt.

Hierbei zeigte die Verbindung der Formel I eine sehr gute Hemmung des cytopathischen Effektes, welche derjenigen, die mit der Vergleichsverbindung bei gleicher Konzentration erzielt wurde, deutlich überlegen war. Auch die ermittelten Virustiter waren nach Applikation des erfindungsgemäß zu verwendenden Wirkstoffes deutlich niedriger als im Falle der Vergleichsverbindung.

Beispiel 2

Test antiviraler Wirkung im Meerschweinchen-cutan-Infektionsmodell

Diese Versuche wurden im wesentlichen nach dem Protokoll von Huber et al (J. Invest. Dermatol. 62, 92-95, 1974) durchgeführt.

400 -500 g schwere Meerschweinchen wurden im Bereich der Hinterflanke enthaart, narkotisiert (Nembutal) und mit einem multiplen Nadelschußapparat (nach Buntscheidt) mit HSV-1 WAL (ca. 1 X $10^5$ bis 5 X $10^5$ PFU/Tier) intracutan infiziert. Die zu testenden Substanzen wurden peroral zu verschiedenen Zeiten (2 -48 h) nach der Infektion mit der Schlundsonde appliziert. Die Ausbildung und das Abheilen von viralbedingten Läsionen an den Infektionsstellen wurden verfolgt und fotographisch dokumentiert.

Während die Applikation der Vergleichsverbindung einen gegenüber unbehandelten Kontrollen nicht signifikanten Einfluß auf den Infektionsverlauf zeigte, bewirkte die Verbindung gemäß Formel I in derselben Dosierungsmenge eine geringere Ausbildung sowie ein rascheres Abheilen dieser viral bedingten Läsionen.

Die Verbindung I ist daher besonders geeignet zur enteralen, parenteralen und äußerlichen Behandlung von Virusinfektionen an Mensch und Tier.

In der Humanmedizin wären aufgrund der Untersuchungen folgende Indikationen als Beispiele zu nennen: Herpes labialis und Herpes genitalis, Herpes Zoster - (Gürtelrose), Varizellen (Windpocken), Keratoconjunctivitis herpetica, infektiöse Mononucleose und Cytomegalovirus-Infektionen.

In der Tiermedizin kommen Pseudorabies-Virus-Infektionen von Schwein und Rind, Rhinotracheritis-Virus-Infektionen des Pferdes sowie die Marek'sche Krankheit bei Hühnern als Anwendungsgebiete in Frage.

Die Verbindung I kann allein oder zusammen mit anderen bekannten Wirkstoffen, insbesondere Antibiotika, verwendet werden.

Die Herstellung der chemotherapeutischen Mittel oder Zubereitungen mit üblichen festen, halbfesten oder flüssigen Trägerstoffen oder Verdünnungsmitteln und den üblicherweise verwendeten galenischen Hilfsstoffen entsprechend der gewünschten Applikationsart mit einer zur Anwendung geeigneten Dosierung erfolgt in üblicher Weise, insbesondere durch Vermischen (vgl. H. Sucker et al, Pharmazeutische Technologie, Thieme-Verlag. Stuttgart, 1978).

Als Darreichungsformen kommen beispielsweise Tabletten, Dragees, Kapseln, Pillen, Suppositorien, wäßrige Lösungen, Suspensionen und Emulsionen, gegebenenfalls sterile injizierbare Lösungen, nichtwäßrige Emulsionen, Suspensionen und Lösungen, Salben, Cremes, Pasten, Lotions, Pulver etc. in Betracht.

Die Verbindung I ist in pharmazeutischen Zubereitungen vorzugsweise in einer Konzentration von 0,5 bis 90 Gewichtsprozent der Gesamtmischung vorhanden.

Im allgemeinen kann bei oraler Verabfolgung sowohl in der Human-als auch in der Veterinärmedizin der Wirkstoff in Mengen von etwa 0,1 bis etwa 10,0, vorzugsweise 0,2 bis 6 mg/kg Körpergewicht pro Tag, vorzugsweise in Form mehrerer Einzelgaben zur Erzielung der gewünschten Ergebnisse verabreicht werden. Es kann jedoch erforderlich sein, von den genanten Dosierungen abzuweichen, und zwar in Abhängigkeit von der Art und der Schwere der Erkrankung, der Art der Zubereitung und der Applikation des Arzneimittels, sowie dem Zeitraum bzw. Intervall, innerhalb welchem die Verabreichung erfolgt. So kann es in

einigen Fällen ausreichend sein, mit weniger als der obengenannten Menge Wirkstoff auszukommen, während in anderen Fällen die oben angeführte Wirkstoffmenge überschritten werden muß.

Beispiele für pharmazeutische Zubereitungen:

Beispiel A

Tablette mit 250 mg Wirkstoff Zusammensetzung für 1000 Tabletten:

Verbindung der Formel I 250 g

Kartoffelstärke 100 g

Milchzucker 50 g

Gelatinelösung 4 % 45 g

Talkum 10 g

Herstellung

Der fein gepulverte Wirkstoff, Kartoffelstärke und Milchzucker werden gemischt. Die Mischung wird mit ca. 45 g 4 % Gelatinelösung durchfeuchtet, feinkörnig granuliert und getrocknet. Das trockene Granulat wird gesiebt, mit 10 g Talkum vermischt und auf einer Rundläufer-Tablettiermaschine zu Tabletten verpreßt. Die Tabletten werden in dicht schließende Behälter aus Polypropylen gefüllt.

Beispiel B

Creme aus 1 % Wirkstoff:

Verbindung der Formel I 1,0 g

Glycerinmonostearat 10,0 g

Cetylalkohol 4,0 g

Polyethylenglykol-400-stearat 10,0 g

Polyethylenglykol-sorbitanmonostearat 10,0 g

Propylenglykol 6,0 g

p-Hydroxybenzoesäuremethylester 0,2 g

Entmineralisiertes Wasser ad 100,0 g

Herstellung:

Der feinst gepulverte Wirkstoff wird mit Propylenglykol suspendiert und die Suspension in die auf 65°C erwärmte Schmelze aus Glycerinmonostearat, Cetylalkohol, Polyethylenglykol-400-stearat und Polyethylenglykolsorbitanmonostearat gerührt. In diese Mischung wird die 70°C heiße Lösung des p-Hydroxybenzoesäuremethylesters in Wasser emulgiert. Nach dem Erkalten wird die Creme über eine Kolloidmühle homogenisiert und in Tuben abgefüllt.

Beispiel C

Puder mit 1 % Wirkstoff:

Verbindung der Formel I 1,0 g

Zinkoxid 10,0 g

Magnesiumoxid 10,0 g

Hochdisperses Siliciumdioxid 2,5 g

Magnesiumstearat 1,0 g

Talkum 75,5 g

Herstellung

Der Wirkstoff wird auf einer Luftstrahlmühle mikronisiert und mit den anderen Bestandteilen homogen vermischt. Die Mischung wird durch ein Sieb (Maschenweite Nr. 7) geschlagen und in Polyethylenbehälter mit Streueinsatz abgefüllt.

**Ansprüche**

1. Verwendung von 1-(4-Chlorphenyl)-1-(2,4-dichlorphenyl)-2-(1,2,4-triazol-1-yl)-ethan-1-ol (Formel I)

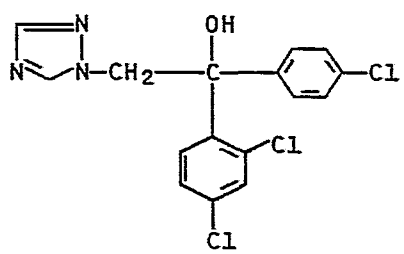

(I)

sowie seiner physiologisch verträglichen Salze bei der Herstellung von Arzneimitteln gegen Viruserkrankungen.

2. Verfahren zur Herstellung von Arzneimitteln zur enteralen, parenteralen oder topischen Behandlung von Virusinfektionen bei Mensch und Tier, dadurch gekennzeichnet, daß man eine wirksame Menge 1-(4-Chlorphenyl)-1-(2,4-dichlorphenyl)-2-(1,2,4-triazol-1-yl)-ethan-1-ol oder eines seiner physiologisch verträglichen Salze in üblicher Weise mit mindestens einem üblichen galenischen Hilfsmittel zubereitet.